(19) Europäisches Patentamt
European Patent Office
Office européen des brevets

(11) Veröffentlichungsnummer: **0 043 967**
**B1**

(12) # EUROPÄISCHE PATENTSCHRIFT

(45) Veröffentlichungstag der Patentschrift: **16.03.83**

(51) Int. Cl.³: **C 07 D 493/08** // A61K31/335, C07D311/94 ,(C07D493/08, 319/00, 311/00)

(21) Anmeldenummer: **81105007.9**

(22) Anmeldetag: **27.06.81**

(54) **2,9-Dioxatricyclo(4,3,1,0(3,7))decane.**

(30) Priorität: **14.07.80 DE 3026579**

(43) Veröffentlichungstag der Anmeldung: **20.01.82 Patentblatt 82/3**

(45) Bekanntmachung des Hinweises auf die Patenterteilung: **16.03.83 Patentblatt 83/11**

(84) Benannte Vertragsstaaten: **AT BE CH DE FR GB IT LI LU NL SE**

(56) Entgegenhaltungen:
**TETRAHEDRON LETTERS, Nr. 35, Juli 1970, Seiten 3087-3090, Oxford, G.B., P. W. THIES: «Stereochemie des Didrovaltratum und Synthese einiger 2,9-Dioxatricyclo (4, 3, 1, 03, 7) Decane»**

**Die Akte enthält technische Angaben, die nach dem Eingang der Anmeldung eingereicht wurden und die nicht in dieser Patentschrift enthalten sind.**

(73) Patentinhaber: **Kali-Chemie Pharma GmbH, Hans-Böckler-Allee 20 Postfach 220, D-3000 Hannover 1 (DE)**

(72) Erfinder: **Thies, Peter Willibrord, Dr. phil., Ihmeplatz 6, D-3000 Hannover 91 (DE)**
Erfinder: **David, Samuel, Dr. rer.nat., Dipl.-Chem., Tiergartenstrasse 103 c, D-3000 Hannover 73 (DE)**

(74) Vertreter: **Lauer, Dieter, Dr., c/o Kali-Chemie Aktiengesellschaft Postfach 220, D-3000 Hannover 1 (DE)**

**EP 0 043 967 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# 2,9-Dioxatricyclo[4,3,1,0(3,7)]decane

Die Erfindung betrifft 3β-Hydroxymethyl-4α-hydroxy-8β-alkoxy-2,9-dioxatricyclo[4,3,1,$0^{3,7}$]decane der allgemeinen Formel I

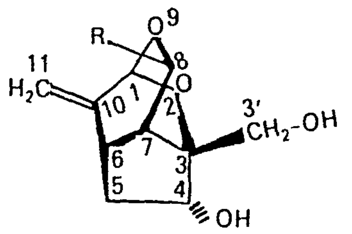

(I)

in der R einen Alkoxyrest mit 1 bis 4 Kohlenstoffatomen bedeutet und die 10,11-Doppelbindung auch zur β-ständigen Methylgruppe hydriert sein kann.

Beispiele für erfindungsgemässe 2,9-Dioxatricyclo[4,3,1,$0^{3,7}$]decane sind vorzugsweise

3β-Hydroxymethyl-4α-hydroxy-8β-methoxy-10-methylen-2,9-dioxatricyclo[4,3,1,$0^{3,7}$]decan,
3β-Hydroxymethyl-4α-hydroxy-8β-methoxy-10β-methyl-2,9-dioxatricyclo[4,3,1,$0^{3,7}$]decan,
3β-Hydroxymethyl-4α-hydroxy-8β-äthoxy-10-methylen-2,9-dioxatricyclo[4,3,1,$0^{3,7}$]decan,
3β-Hydroxymethyl-4α-hydroxy-8β-äthoxy-10β-methyl-2,9-dioxatricyclo[4,3,1,$0^{3,7}$]decan und
3β-Hydroxymethyl-4α-hydroxy-8β-butoxy-10β-methyl-2,9- dioxatricyclo[4,3,1,$0^{3,7}$]decan.

In einer Reihe älterer Anmeldungen sind bereits 2,9-Dioxatricyclo[4,3,1,$0^{3,7}$]decane beschrieben, die sich durch vorteilhafte pharmakologische Eigenschaften, insbesondere durch neuartige Wirkungen auf das zentrale Nervensystem auszeichnen, darunter auch solche mit schlaffördernden Eigenschaften (DE-OS 26 07 106). Ursächlich für die Wirkungen auf den Schlaf, die sich in einer Vermehrung des Tiefschlafs und auch des Paradoxalschlafs zeigen, scheint dabei im Vergleich mit den anderen bekannten 2,9-Dioxatricyclo[4,3,1,$0^{3,7}$]decanen, die in 3-Stellung des 2,9-Dioxatricyclo[4,3,1,$0^{3,7}$]decan-Gerüsts eine Methyl- oder Halogenmethyl- oder Azidomethyl-Gruppe haben, die 3-Aminomethyl-Gruppe zu sein.

Der Erfindung liegt nun die Aufgabe zugrunde, neue 2,9-Dioxatricyclo[4,3,1,$0^{3,7}$]decane mit verbesserten pharmakologischen Eigenschaften, insbesondere mit vorteilhaftem Wirkungsprofil, vorzugsweise mit einschlaffördernden Wirkungen zu schaffen.

Überraschenderweise wurde bei der Lösung der Aufgabe gefunden, dass durch Ersatz der bekannten 3-Methyl- bzw. 3-Halogenmethyl- bzw. 3-Azidomethyl- bzw. 3-Aminomethyl-Gruppe durch eine 3-Hydroxymethyl-Gruppe 2,9-Dioxatricyclo[4,3,1,$0^{3,7}$]decane erhalten werden, die die gewünschten Eigenschaften zeigen.

Gegenstand der Erfindung sind demnach die eingangs und in den Ansprüchen genannten neuen 2,9-Dioxatricyclo[4,3,1,$0^{3,7}$]decane.

Die erfindungsgemässen Verbindungen hemmen die motorische Aktivität und zeigen am EEG der Ratte deutlich eine einschlaffördernde Wirkung. In den ersten 3 Stunden nach Applikation zeigen diese Verbindungen eine stärkere wachvermindernde Wirkung als in den nachfolgenden 5 Stunden, so dass die Gefahr eines «hang over» beim Menschen gering ist.

So lässt z.B. das erfindungsgemässe 3β-Hydroxymethyl-4α-hydroxy-8β-methoxy-10β-methyl-2,9-dioxatricyclo[4,3,1,$0^{3,7}$]decan bei Mäusen im Lichtschrankenkäfig bereits bei 0,8 mg/kg eine Motilitätshemmung erkennen. Bei der Ratte beträgt die Dosis für die Einschlafförderung laut 3-Stunden-EEG ca. 2,5 mg/kg. Die Toxizität ist mit einer nach i.p.-Applikation an der Maus ermittelten $LD_{50}$ von grösser als 1600 mg/kg extrem günstig.

Die innovative Struktur der erfindungsgemässen Verbindungen, ihre starke Wirkung als «Schlaf-Inducer» sowie die gute Verträglichkeit entsprechen einem grossen Bedarf an neuartigen Schlafmitteln.

Die Herstellung der erfindungsgemässen Verbindungen konnte in stereochemisch hochselektiven Verfahren in mehreren Varianten verwirklicht werden. Die Verfahren werden anhand des nachfolgenden Formelschemas näher erläutert.

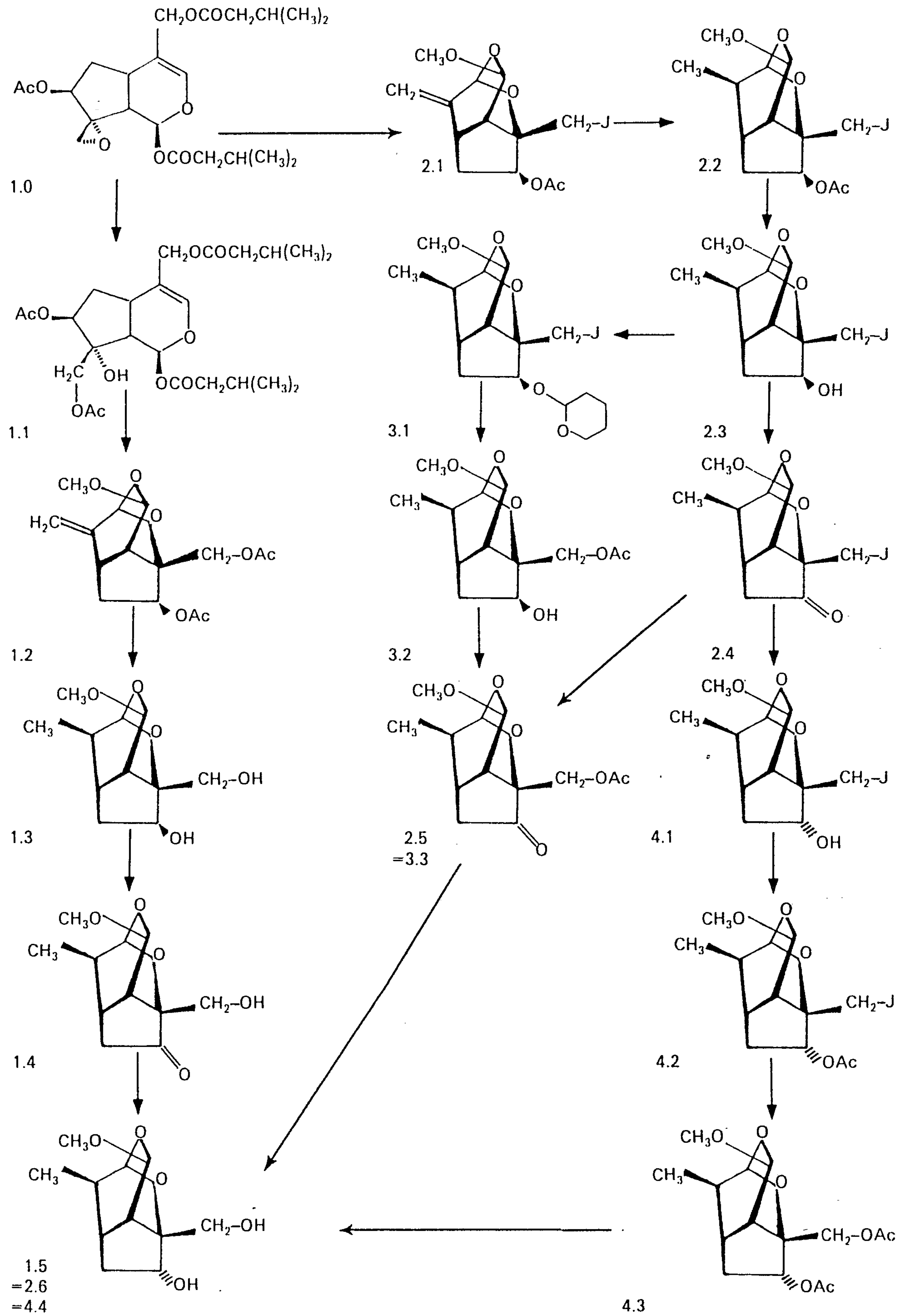

1.0
1.1
1.2
1.3
1.4
1.5
=2.6
=4.4
2.1
2.2
2.3
2.4
2.5
=3.3
3.1
3.2
4.1
4.2
4.3

Dem Formelschema liegt die 8β-Methoxy-10β-Methyl-Verbindung zugrunde. Es versteht sich dabei, dass die beschriebenen Verfahren nicht auf diese Verbindung beschränkt sind, sondern entsprechend auch für die anderen erfindungsgemässen Verbindungen gelten, wie das durch die Erläuterungen und die beigefügten Beispiele offenbart wird.

Ausgangsprodukt ist in allen Fällen Didrovaltratum (1-Isovaleroxy-4-isovaleroxymethyl-7-acetoxy-8-spirooxirano-1,9-dihydrocyclopenta(c)pyran) bzw. ein Gesamtextrakt aus Valerianaceen mit etwa 70% Gehalt an Didrovaltratum. Gemäss Variante 1 wird Didrovaltratum (1.0) mittels Eisessig/Acetanhydrid in das entsprechende Acetohydrin (1.1) überführt, welches anschliessend mittels Methanol/p-Toluolsulfonsäure zum 3β-Acetoxymethyl-4β-acetoxy-8β-methoxy-10-methylen-2,9-dioxatricyclo[4,3,1,$0^{3,7}$]decan (1.2) umgesetzt wird.

Diese beiden Verfahrensstufen sind an sich in der DE-OS 19 61 433 beschrieben. Verwendet man statt des erwähnten Methanol andere Alkohole, dann werden je nach verwendetem Alkohol die entsprechenden 8-Alkoxy-Verbindungen erhalten, wie das aus der DE-OS 19 61 433 an sich bekannt ist.

Nach Verseifung der beiden Acetoxygruppen, beispielsweise mittels wässriger oder wässrig-alkoholischer Alkalihydroxidlösung oder Kaliumcarbonat/Methanol bzw. falls als Endprodukt - wie im Formelschema dargestellt - die 10β-Methyl-Verbindung gewünscht wird, nach Hydrierung der 10,11-Doppelbindung, beispielsweise mittels Wasserstoff an Raney-Nickel in Gegenwart einer starken anorganischen Base, wobei gleichzeitig die beiden Acetoxygruppen verseift werden, wird die Dihydroxy-Verbindung (1.3) erhalten.

Anschliessend wird die 4β-Hydroxygruppe selektiv, beispielsweise mittels Chromtrioxid, bevorzugt mittels Pyridinchlorochromat in das entsprechende Keton (1.4) oxidiert. Schliesslich wird die 4-on-Verbindung mittels komplexer Metallhydride, z.B. mittels Lithiumaluminiumhydrid, bevorzugt mittels Natriumborhydrid selektiv zum gewünschten 4α-Hydroxy-Endprodukt (1.5) reduziert.

Gemäss Variante 2 wird Didrovaltratum (1.0) mittels Methanol/Jodwasserstoffsäure, wie aus der DE-OS 21 29 507 an sich bekannt, direkt zum 3β-Jodomethyl-4β-acetoxy-8β-Methoxy-2,9-dioxatricyclo[4,3,1,$0^{3,7}$]decan (2.1) umgesetzt, wobei - wie in der DE-OS 21 29 507 bereits beschrieben - je nach verwendetem Alkohol und je nach verwendeter Halogenwasserstoffsäure entsprechende 8β-Alkoxy- bzw. 3β-Halogenmethyl-Verbindungen erhalten werden können.

Je nachdem, ob als Endprodukt die 10β-Methyl- oder die 10-Methylen-Verbindung gewünscht wird, wird die 10,11-Doppelbindung mittels Wasserstoff an Platinoxid, wie aus der DE-OS 27 19 916 an sich bekannt, zur β-ständigen Methylgruppe hydriert und entweder die erhaltene 10β-Methyl-Verbindung (2.2) oder gleich die 10-Methylen-Verbindung (2.1) beispielsweise mittels wässriger oder wässrig-alkoholischer Alkalihydroxidlösung oder Kaliumcarbonat/Methanol zur 3β-Jodomethyl-4β-hydroxy-Verbindung (2.3) verseift. Diese wird dann, beispielsweise mittels Jones-Reagenz, bevorzugt mittels Pyridinchlorochromat, zum Decan-4-on-(2.4) oxidiert. Anschliessend wird die 3β-Jodomethyl-Gruppe der Verbindung 2.4, beispielsweise mittels Natrium- oder Kaliumacetat, bevorzugt in Gegenwart von quartären Alkylammoniumacetaten wie z.B. Tetraäthyl- oder Tetrabutyl-Ammoniumacetat in die 3β-Acetoxy-Gruppe der Verbindung 2.5 umgewandelt. Schliesslich wird die 4-on-Funktion in der erhaltenen 3β-Acetoxymethyl-Verbindung (2.5) mittels komplexer Metallhydride, wie beispielsweise Lithiumaluminiumhydrid, zur 4α-Hydroxyfunktion reduziert, wobei im Fall der Reduktion mittels Lithiumaluminiumhydrid gleichzeitig die 3β-Acetoxymethyl-Gruppe zur 3β-Hydroxymethyl-Gruppe reduziert und so das gewünschte Dihydroxy-Endprodukt (2.6 = 1.5) erhalten wird. Bei Reduktion der 4-on-Gruppe mit anderen komplexen Metallhydriden, z.B. Natriumborhydrid, wird anschliessend an die Reduktion die 3β-Acetoxymethylgruppe zur 3β-Hydroxymethylgruppe verseift.

Gemäss Variante 3 wird die 4β-Hydroxy-Funktion der Verbindung 2.3 zunächst durch Umsetzung mit 3,4-Dihydro-2H-pyran zur 4-(2'-Tetrahydropyranyloxy)-Verbindung 3.1 geschützt. Nach Austausch der Halogenfunktion durch die Acetoxyfunktion in der bereits beschriebenen Weise, wird die Schutzgruppe wieder entfernt, z.B. mittels wässriger Salzsäure. Die nun wieder freie 4β-Hydroxy-Funktion in der erhaltenen Verbindung 3.2 kann, wie oben bereits beschrieben, zur 4-on-Funktion oxidiert werden, wobei man die Verbindung 2.5 erhält, die in der beschriebenen Weise zum Endprodukt 2.6 = 1.5 reduziert und verseift wird.

Gemäss Variante 4 wird die 3-Jodomethyl-4-on-Verbindung 2.4 selektiv mittels komplexer Metallhydride, bevorzugt mittels Natriumborhydrid, zur 4α-Hydroxy-Verbindung 4.1 reduziert. Anschliessend wird diese zur 4α-Acetoxy-Verbindung 4.2 acetyliert und in dieser die Halogenfunktion der 3β-Jodomethylgruppe in der bereits mehrfach beschriebenen Weise gegen die Acetoxy-Funktion ausgetauscht, so dass man das Diacetat 4.3 erhält. Durch Verseifung der beiden Acetoxy-Gruppen erhält man dann das gewünschte Diol 1.5.

Die nachfolgenden Beispiele dienen der näheren Erläuterung der Erfindung. Der Einfachheit halber ist in der Beschreibung der Beispiele der Begriff «Dioxatricyclo[4,3,1,$0^{3,7}$]decan» mit «DTD» abgekürzt. Die Angabe «Fp <0°C» bedeutet, dass es sich um eine bei Zimmertemperatur ölige Verbindung handelt.

Beispiel 1

1.1 Didrovaltratum-acetoxy-hydrin

a) aus Valerianaceen-Extrakt

424 g eines Extrakts aus Valerianaceen mit etwa

70% Didrovaltratum-Gehalt wurden in 310 ml Eisessig und 31 ml Acetanhydrid bei 80°C gelöst. Nach Abkühlung auf Raumtemperatur wurden 660 ml Triäthylamin zugesetzt. Der Ansatz wurde dann 2½ Stunden bei 80°C gerührt. Nach Beendigung der Reaktion wurde mit Wasser verdünnt und mit Äther extrahiert. Die vereinten organischen Phasen wurden über $Na_2SO_4$ getrocknet, filtriert und im Vakuum eingeengt.

Ausbeute: 336 g ≙ 70% d.Th., bezogen auf 100%iges Didrovaltratum

| | |
|---|---|
| $C_{24}H_{36}O_{10}$ | Fp.: <0°C |
| MG: 484,55 | $[\alpha]_D^{20}$ : −38° (MeOH) |

b) aus Didrovaltratum

10 g Didrovaltratum wurden in 11 ml Eisessig und 1,1 ml Essigsäureanhydrid bei 80°C gelöst. Der Ansatz wurde auf 20°C abgekühlt und mit 25 ml Triäthylamin versetzt. Die Lösung wurde anschliessend 2½ Stunden auf 80°C erhitzt. Dann wurde die Lösung auf Eiswasser gegeben und mit Äther extrahiert. Die vereinten organischen Phasen wurden über $Na_2SO_4$ getrocknet, filtriert und im Vakuum eingeengt.

Rohausbeute: 9,8 g ≙ 85,9% d.Th.
Das Produkt ist identisch mit dem nach Beispiel 1.1.a erhaltenen Produkt.

1.2 3β-Acetoxymethyl-4β-acetoxy-8β-methoxy-10-methylen-2,9-DTD

336 g (1.1.a), gelöst in 690 ml Methanol, wurden mit 6,9 g p-Toluolsulfonsäure (pTS) versetzt und 30 Minuten auf 60°C erwärmt. Anschliessend wurde die Lösung auf Eiswasser gegeben und mit Äther extrahiert. Die vereinten organischen Phasen wurden über $Na_2SO_4$ getrocknet, filtriert und im Vakuum eingeengt.

Ausbeute: 211 g ≙ 66% d.Th., bezogen auf 100%iges Didrovaltratum

| | |
|---|---|
| $C_{15}H_{20}O_7$ | Fp.: <0°C |
| MG: 312.35 | $[\alpha]_D^{20}$ : +45° (MeOH) |

1.3 3β-Hydroxymethyl-4β-hydroxy-8β-methoxy-10β-methyl-2,9-DTD

71,7 g (1.2) wurden in Methanol gelöst und nach Zugabe von 24,0 g NaOH (in Methanol) und Raney-Nickel bei Raumtemperatur hydriert. Anschliessend wurde vom Katalysator abfiltriert und nach Neutralisieren mit Essigsäure im Vakuum eingeengt. Der Rückstand wurde in Wasser aufgenommen und mit Äther extrahiert. Die vereinten organischen Phasen wurden über $Na_2SO_4$ getrocknet, filtriert und im Vakuum eingeengt.

Ausbeute: 42,4 g ≙ 80.2% d.Th.

| | |
|---|---|
| $C_{11}H_{18}O_5$ | Fp.: 89–90°C |
| MG: 230.26 | $[\alpha]_D^{20}$ : −32° (MeOH) |

1.4 3β-Hydroxymethyl-8β-methoxy-10β-methyl-2,9-DTD-4-on

30 g (1.3), in Dichlormethan gelöst, wurden unter Rühren zu einer Suspension von 37 g Pyridinchlorochromat in Dichlormethan zugetropft. Nach 4 Stunden wurden 2,5 l Äther hinzugegeben und die Lösung von den ausgefallenen Salzen abfiltriert. Nach Abdestillieren des Lösungsmittels wurde der Rückstand über Kieselgel mittels Äther/n-Hexan gereinigt.

Ausbeute: 17,8 g ≙ 60% d.Th.

| | |
|---|---|
| $C_{11}H_{16}O_5$ | Fp.: 97–104°C |
| MG: 228.27 | $[\alpha]_D^{20}$ : −61,2° (MeOH) |

1.5 3β-Hydroxymethyl-4α-hydroxy-8β-methoxy-10β-methyl-2,9-DTD

1,5 g (1.4), in 40 ml Äthanol gelöst, wurden unter Rühren mit 0,5 g $NaBH_4$ versetzt. Nach 1 Stunde wurde der Ansatz mit verdünnter HCl neutralisiert und anschliessend mit Äther extrahiert. Die vereinten organischen Phasen wurden über $Na_2SO_4$ getrocknet, filtriert und im Vakuum eingeengt.

Ausbeute: 1,35 g ≙ 89.6% d.Th.

| | |
|---|---|
| $C_{11}H_{18}O_5$ | Fp.: 66–70°C |
| MG: 239.26 | $[\alpha]_D^{20}$ : −57° (MeOH) |

Beispiel 2

2.1 3β-Jodomethyl-4β-acetoxy-8β-methoxy-10-methylen-2,9-DTD

850 g eine Extrakts aus Valerianaceen, welcher zu etwa 70% aus Didrovaltratum bestand, wurden in 1 Liter Methanol gelöst. Die homogene Lösung wurde anschliessend langsam mit 220 ml 57%iger Jodwasserstoffsäure, gelöst in einem Liter Methanol, bei 20°C versetzt. Anschliessend wurde 2 Stunden bei 60°C unter Rühren stehengelassen.

Danach wurde in kleinen Anteilen mit insgesamt einem Liter n-Hexan zur Entfernung lipophiler Anteile extrahiert. Die Hexanphasen wurden verworfen. Dann wurde der Ansatz mit 5 Liter Wasser verdünnt und viermal mit je 3 Liter Äther extrahiert. Die Wasserphase wurde verworfen, die Ätherphase mit insgesamt 5 Liter Wasser gewaschen, mit Natriumbicarbonatlösung neutralisiert und über Natriumsulfat und Kohle getrocknet bzw. geklärt. Nach Filtration wurde das Filtrat im Vakuum bis zur Gewichtskonstanz eingeengt. Es wurden 565 g eines gelbgefärbten Öles erhalten, aus dem 316,9 g 3β-Jodomethyl-4β-acetoxy-8β-methoxy-10-methylen-2,9-dioxatricyclo-$[4,3,1,0^{3,7}]$decan auskristallisierten. Das sind, bezogen auf eingesetztes Didrovaltratum, 59,5% der theoretisch möglichen Menge.

| | |
|---|---|
| $C_{13}H_{17}O_5J$ | Fp.: 104–106°C |
| MG: 380,19 | $[\alpha]_D^{20}$ : +68° (MeOH) |

2.2 3β-Jodomethyl-4β-acetoxy-8β-methoxy-10β-methyl-2,9-DTD

Eine Lösung von 800 g (2.1) in 3 l Essigester wurde zu einer Suspension von 35 g vorhydriertem Platinoxid in 300 ml Essigester gegeben. Es wurde bei Raumtemperatur und Normaldruck hydriert. Die Wasserstoffaufnahme war anfangs sehr schnell, gegen Ende verlief sie jedoch sehr langsam. Nach Aufnahme der theoretischen Menge an Wasserstoff (47,2 l) wurde die Reaktionsmischung unter Stickstoff-Atmosphäre über Asbest filtriert. Nach Einengen erhielt man 804 g Rohprodukt (≙ 100% d.Th.). Nach mehrmaligem Umkristallisieren aus Methanol erhielt man 542 g reines 10β-Epimeres (2.2) (≙ 67% d.Th. bezogen auf (2.1)).

| | |
|---|---|
| $C_{13}H_{19}JO_5$ | Fp.: 129°C |
| MG: 382.19 | $[\alpha]_D^{20}$ : −24,5° (MeOH) |

2.3 3β-Jodomethyl-4β-hydroxy-8β-methoxy-10β-methyl-2,9-DTD

100 g (2.2) in 400 ml Methanol wurden mit 18 g $K_2CO_3$ versetzt. Die Suspension wurde 2 Stunden bei Raumtemperatur gerührt, vom überschüssigen $K_2CO_3$ abfiltriert und mit wässriger Essigsäure neutralisiert. Nach Extraktion mit $CH_2Cl_2$ wurden die organischen Phasen vereinigt und über $Na_2SO_4$ getrocknet. Nach Abfiltrieren und Einengen im Vakuum erhielt man 69,37 g (2.3) ≙ 82,6% d.Th.

| | |
|---|---|
| $C_{11}H_{17}O_4J$ | Fp.: 92–93°C |
| MG: 340.166 | $[\alpha]_D^{20}$ : −35,3° (MeOH) |

2.4 3β-Jodomethyl-8β-methoxy-10β-methyl-2,9-DTD-4-on

8 g (2.3) in Dichlormethan wurden zu einer Suspension von 15 g Pyridinchlorochromat in Dichlormethan unter starkem Rühren hinzugetropft. Nach 3 Stunden wurden 400 ml Äther hinzugegeben. Die ausgefallenen Salze wurden abfiltriert und mit Äther gewaschen. Die vereinten organischen Phasen wurden im Vakuum eingeengt und der Rückstand über Kieselgel mittels Äther/n-Hexan gereinigt.

Ausbeute: 6,9 g ≙ 86,8% d.Th.

| | |
|---|---|
| $C_{11}H_{15}O_4J$ | Fp.: <0°C |
| MG: 338.17 | $[\alpha]_D^{20}$ : −28,8° (MeOH) |

2.5 3β-Acetoxymethyl-8β-methoxy-10β-methyl-2,9-DTD-4-on

20 g (2.4) in 100 ml Dimethylformamid wurden mit 20 g Tetraäthylammoniumacetat versetzt und 4 Stunden unter Rühren auf 110° erhitzt. Anschliessend wurde das Lösungsmittel abdestilliert, der Rückstand in Wasser aufgenommen und mit Äther extrahiert. Die vereinten organischen Phasen wurden über $Na_2SO_4$ getrocknet, filtriert und im Vakuum eingeengt.

Ausbeute: 9,5 g ≙ 66% d.Th.

| | |
|---|---|
| $C_{13}H_{18}O_6$ | Fp.: <0°C |
| MG: 270.28 | $[\alpha]_D^{20}$ : −50,9° (MeOH) |

2.6 3β-Hydromethyl-4α-hydroxy-8β-methoxy-10β-methyl-2,9-DTD

9,5 g (2.5) in 100 ml THF abs. wurden zu einer Suspension von 2,8 g $LiALH_4$ in 100 ml THF abs. unter Rühren und unter Stickstoff bei 0°C hinzugetropft. Anschliessend wurde die Lösung noch 1 Stunde bei Raumtemperatur gerührt.

Dann wurden zunächst 50 ml feuchter Äther, später ca. 5 ml Wasser hinzugegeben. Es wurde von den ausgefallenen Salzen abfiltriert und das Filtrat im Vakuum eingeengt. Der Rückstand wurde mit gesättigter Ammoniumsulfatlösung aufgenommen und mit $CH_2Cl_2$ extrahiert. Die vereinten organischen Phasen wurden über $Na_2SO_4$ getrocknet, filtriert und im Vakuum eingeengt.

Ausbeute: 4,1 g ≙ 50,6% d.Th.

Die Verbindung zeigte sich identisch mit der gemäss Beispiel 1 hergestellten Verbindung 1.5.

Beispiel 3

3.1 3β-Jodomethyl-4β-(tetrahydropyranyl-2)-8β-methoxy-10β-methyl-2,9-DTD

43,4 g (2.3) in 300 ml $CH_2Cl_2$ wurden mit 17 ml 3,4-Dihydro-2H-pyran und einer Spatelspitze Pikrinsäure versetzt und 1 Stunde bei Raumtemperatur gerührt. Das Reaktionsgemisch wurde anschliessend zweimal gegen eine gesättigte $NaHCO_3$-Lösung geschüttelt und zweimal mit Wasser gewaschen. Die Dichlormethanphase wurde über $Na_2SO_4$ getrocknet, filtriert und im Vakuum eingeengt.

Ausbeute: 53,15 g ≙ 98.2% d.Th.

| | |
|---|---|
| $C_{16}H_{25}JO_5$ | Fp.: 95–97°C |
| MG: 424.27 | $[\alpha]_D^{20}$ : +35,3° (MeOH) |

3.2 3β-Acetoxymethyl-4β-hydroxy-8β-methoxy-10β-methyl-2,9-DTD

42,4 g (3.1) in 300 ml Dimethylformamid wurden mit 20 g Tetrabutylammoniumacetat und 40 g Kaliumacetat versetzt und unter Rühren 5 Stunden auf 120° erhitzt. Anschliessend wurde das Lösungsmittel im Vakuum abdestilliert und der Rückstand mit $CH_2Cl_2$ aufgenommen. Die organische Lösung wurde 2mal mit Wasser gewaschen, über $Na_2SO_4$ getrocknet, filtriert und im Vakuum eingeengt. Der Rückstand wurde in 200 ml Methanol aufgenommen, mit 100 ml 1n HCl-Lösung versetzt und 2 Stunden bei Raumtemperatur gerührt. Anschliessend wurde die Lösung schonend auf ein Volumen von ca. 100 ml eingeengt. Die wässrige Phase wurde zweimal mit je 100 ml $CH_2Cl_2$ extrahiert. Die vereinten organischen Phasen wurden dann über $Na_2SO_4$ getrocknet, filtriert und im Vakuum eingeengt.

$C_{14}H_{20}O_6$ Fp.: <0°C
MG: 284.31 $[\alpha]_D^{20}$ : −23,9° (MeOH)

3.3 3β-Acetoxy-8β-methoxy-10β-methyl-2,9-DTD-4-on

28,2 g (3.2) in 500 ml Aceton wurden bei 0°C unter Rühren tropfenweise mit 55 ml Jones-Reagenz ($CrO_3$ + $H_2SO_4$) versetzt. Nach beendeter Reaktion wurden 5 ml Isopropanol hinzugegeben und von den Chromsalzen abfiltriert. Die Salze wurden mit 300 ml $CH_2Cl_2$ gewaschen und die vereinten organischen Lösungen mit wässriger $Na_2CO_3$-Lösung neutralisiert. Nach Aussalzen mit $(NH_4)_2SO_4$ wurde mit $CH_2Cl_2$ extrahiert. Die vereinten organischen Phasen wurden über $Na_2SO_4$ getrocknet, filtriert und im Vakuum eingeengt.

Ausbeute: 23,1 g ≙ 81,9% d.Th.

Die erhaltene Verbindung zeigte sich identisch mit der gemäss Beispiel 2.5 hergestellten Verbindung.

Die weitere Umsetzung zum Endprodukt erfolgte gemäss dem Beispiel 2.6.

Beispiel 4

4.1 3β-Jodomethyl-4α-hydroxy-8β-methoxy-10β-methyl-2,9-DTD

Zu 3,3 g Jodketon (2.4) in 20 ml THF abs. gab man unter Stickstoff-Atmosphäre 1,5 g $NaBH_4$, aufgeschlämmt in 10 ml THF abs. Die Reaktion dauerte 1 Stunde und erfolgte bei Raumtemperatur. Anschliessend wurden 40 ml Wasser hinzugegeben und die Lösung mit $CH_2Cl_2$ extrahiert. Die vereinten organischen Phasen wurden über $Na_2SO_4$ getrocknet, filtriert und im Vakuum eingeengt. Es resultierten 3,3 g Rohprodukt. Nach Umkristallisation aus n-Hexan/Äther erhielt man 2,8 g (4.1) ≙ 84,3% d.Th.

$C_{11}H_{17}O_4J$ Fp.: 75–77°C
MG: 340.166 $[\alpha]_D^{20}$ : −18,5° (MeOH)

4.2 3β-Jodomethyl-4α-acetoxy-8β-methoxy-10β-methyl-2,9-DTD

34,0 g (4.1) in 50 ml Essigsäureanhydrid + 5 ml Pyridin wurden 1 Stunde bei 60° gerührt. Anschliessend wurde der Ansatz mehrere Male mit Toluol im Vakuum eingeengt. Der Rückstand wurde in Wasser aufgenommen und mit Äther extrahiert. Die vereinten organischen Phasen wurden über $Na_2SO_4$ getrocknet, filtriert und im Vakuum eingeengt.

Ausbeute: 34,9 g ≙ 91,4% d.Th.

$C_{12}H_{19}O_5J$ Fp.: 109–111°C
MG: 382.2 $[\alpha]_D^{20}$ :−4,5° (MeOH)

4.3 3β-Acetoxymethyl-4α-acetoxy-8β-methoxy-10β-methyl-2,9-DTD

34,9 g (4.2) in 600 ml Dimethylformamid p.a. wurden mit 15 g Tetrabutylammoniumacetat und 50 g Natriumacetat (wasserfrei) versetzt und 7 Stunden unter Rühren auf 150°C erhitzt. Anschliessend wurde das Lösungsmittel im Vakuum abdestilliert und der Rückstand mit Wasser aufgenommen. Nach Extraktion mit Äther wurden die vereinten organischen Phasen über $Na_2SO_4$ getrocknet, filtriert und im Vakuum eingeengt.

Ausbeute: 24,9 g ≙ 86,7% d.Th.

$C_{15}H_{22}O_7$ Fp.: <0°C
MG: 314.346 $[\alpha]_D^{20}$ : −29,3° (MeOH)

4.4 3β-Hydroxymethyl-4α-hydroxy-8β-methoxy-10β-methyl-2,9-DTD

8,7 g (4.3) in 100 ml Äther wurden mit 2,3 g NaOH in 40 ml Methanol versetzt und 1 Stunde bei Raumtemperatur gerührt. Anschliessend wurde die Lösung mit Eisessig auf pH 7 gebracht und im Vakuum eingeengt. Der Rückstand wurde mit gesättigter wässriger Ammoniumsulfatlösung aufgenommen und mit $CH_2Cl_2$ extrahiert. Die vereinten organischen Phasen wurden über $Na_2SO_4$ getrocknet, filtriert und im Vakuum eingeengt.

Rohausbeute: 6,2 g
Nach Umkristallisation aus Äther:Hexan: 5,6 g ≙ 80,4% d.Th.

Die Substanz zeigte sich identisch mit der nach den vorherigen Beispielen hergestellten Substanz 1.5 bzw. 2.6.

Beispiel 5

Gemäss den Beispielen 1.1 bis 1.5 wurden unter Ersatz des Methanol durch Äthanol in Beispiel 1.2 die entsprechenden 8β-Äthoxy-Verbindungen hergestellt:

5.1 3β-Acetoxymethyl-4β-acetoxy-8β-äthoxy-10-methylen-2,9-DTD

$C_{16}H_{22}O_7$ Fp.: <0°C
MG: 326.35 $[\alpha]_D^{20}$ : +9,4° (MeOH)

5.2 3β-Hydroxymethyl-4β-hydroxy-8β-äthoxy-10β-methyl-2,9-DTD

$C_{12}H_{20}O_5$ Fp.: 56–57°C
MG: 244.29 $[\alpha]_D^{20}$ : −26,0° (MeOH)

5.3 3β-Hydroxymethyl-8β-äthoxy-10β-methyl-2,9-DTD-4-on

$C_{12}H_{18}O_5$ Fp.: <0°C
MG: 242.2 $[\alpha]_D^{20}$ : −55,6° (MeOH)

5.4 3β-Hydroxymethyl-4α-hydroxy-8β-äthoxy-10β-methyl-2,9-DTD

$C_{12}H_{20}O_5$ Fp.: 86–87°C
MG: 244.29 $[\alpha]_D^{20}$ : −56,3° (MeOH)

Beispiel 6

Gemäss den Beispielen 2.1 und 2.3 bis 2.6 wurden unter Ersatz des Methanol durch Äthanol in Beispiel 2.1 und unter Wegfall der Hydrierung gemäss Beispiel 2.2 die entsprechenden 8β-Äthoxy-10-methylen-Verbindungen hergestellt:

6.1 3β-Jodomethyl-4β-acetoxy-8β-äthoxy-10-methylen-2,9-DTD

$C_{14}H_{19}O_5J$ Fp.: 63–65°C
MG: 394.21 $[\alpha]_D^{20}$ : +76° (MeOH)

6.2 3β-Jodomethyl-4β-hydroxy-8β-äthoxy-10-methylen-2,9-DTD

$C_{12}H_{17}O_4J$ Fp.: <0°C
MG: 352.17 $[\alpha]_D^{20}$ : +18,0° (MeOH)

6.3 3β-Jodomethyl-8β-äthoxy-10-methylen-2,9-DTD-4-on

$C_{12}H_{15}O_4J$ Fp.: <0°C
MG: 350.16 $[\alpha]_D^{20}$ : −3,3° (MeOH)

6.4 3β-Acetoxymethyl-8β-äthoxy-10-methylen-2,9-DTD-4-on

$C_{14}H_{18}O_6$ Fp.: <0°C
MG: 282.29 $[\alpha]_D^{20}$ : −8,0° (MeOH)

6.5 3β-Hydromethyl-4α-hydroxy-8β-äthoxy-10-methylen-2,9-DTD

$C_{12}H_{18}O_6$ Fp.: <0°C
MG: 242.27 $[\alpha]_D^{20}$ : +25,9° (MeOH)

Beispiel 7

Unter Benutzung der Reaktionsfolge gemäss den Beispielen 2.1 bis 2.4 und 4.1 bis 4.4, jedoch unter Ersatz des Methanol durch Äthanol in Beispiel 2.1 wurden die entsprechenden 8β-Äthoxy-10β-methyl-Verbindungen hergestellt:

7.1 3β-Jodomethyl-4β-acetoxy-8β-äthoxy-10-methylen-2,9-DTD

$C_{14}H_{19}O_5J$ Fp.: 63–65°C
MG: 394.21 $[\alpha]_D^{20}$ : +76° (MeOH)

7.2 3β-Jodomethyl-4β-acetoxy-8β-äthoxy-10β-methyl-2,9-DTD

$C_{14}H_{21}O_5J$ Fp.: 103–105°C
MG: 369.228 $[\alpha]_D^{20}$ : +20,7° (MeOH)

7.3 3β-Jodomethyl-4β-hydroxy-8β-äthoxy-10β-methyl-2,9-DTD

$C_{12}H_{18}O_4J$ Fp.: 83-85°C
MG: 353.18 $[\alpha]_D^{20}$ : −41,2° (MeOH)

7.4 3β-Jodomethyl-8β-äthoxy-10β-methyl-2,9-DTD-4-on

$C_{12}H_{16}O_4J$ Fp.: 58–59°C
MG: 351.173 $[\alpha]_D^{20}$ : −32,1° (MeOH)

7.5 3β-Jodomethyl-4α-hydroxy-8β-äthoxy-10β-methyl-2,9-DTD

$C_{12}H_{18}O_4J$ Fp.: <0°C
MG: 353.18 $[\alpha]_D^{20}$ : −16,7° (MeOH)

7.6 3β-Jodomethyl-4α-acetoxy-8β-äthoxy-10β-methyl-2,9-DTD

$C_{14}H_{20}O_5J$ Fp.: <0°C
MG: 395.218 $[\alpha]_D^{20}$ : −6,5° (MeOH)

7.7 3β-Acetoxymethyl-4α-acetoxy-8β-äthoxy-10β-methyl-2,9-DTD

$C_{16}H_{24}O_7$ Fp.: 70–72°C
MG: 328.36 $[\alpha]_D^{20}$ : +27,5° (MeOH)

7.8 = 5.4 3β-Hydroxymethyl-4α-hydroxy-8β-äthoxy-10β-methyl-2,9-DTD

$C_{12}H_{20}O_5$ Fp.: 86–87°C
MG: 244.29 $[\alpha]_D^{20}$ : −56,3°

Beispiel 8

Unter Benutzung der Reaktionsfolge gemäss den Beispielen 2.1, 2.3, 2.4 und 4.1 bis 4.4, also unter Wegfall der Hydrierung gemäss Beispiel 2.2, wurden die entsprechenden 8β-Methoxy-10-methylen-Verbindungen hergestellt:

8.1 = 2.1 3β-Jodomethyl-4β-acetoxy-8β-methoxy-10-methylen-2,9-DTD

$C_{13}H_{17}O_5J$ Fp.: 104–106°C
MG: 380.19 $[\alpha]_D^{20}$ : +68° (MeOH)

8.2 3β-Jodomethyl-4β-hydroxy-8β-methoxy-10-methylen-2,9-DTD

$C_{11}H_{15}O_4J$ Fp.: <0°C
MG: 338.25 $[\alpha]_D^{20}$ : +13° (MeOH)

8.3 3β-Jodomethyl-8β-methoxy-10-methylen-2,9-DTD-4-on

$C_{11}H_{13}O_4J$ Fp.: <0°C
MG: 336.1

8.4 3β-Jodomethyl-4α-hydroxy-8β-methoxy-10-methylen-2,9-DTD

$C_{11}H_{15}O_4J$ Fp.: 108–110°C
MG: 338.15 $[\alpha]_D^{20}$ : +10° (MeOH)

8.5 3β-Jodomethyl-4α-acetoxy-8β-methoxy-10-methylen-2,9-DTD

$C_{13}H_{17}O_5J$ Fp.: 122–124°C
MG: 380.17 $[\alpha]_D^{20}$ : +28,7° (MeOH)

8.6 3β-Acetoxymethyl-4α-acetoxy-8β-methoxy-10-methylen-2,9-DTD

$C_{15}H_{20}O_7$ Fp.: 83–85°C
MG: 312.33 $[\alpha]_D^{20}$ : +24,6° (MeOH)

8.7 3β-Hydroxymethyl-4α-hydroxy-8β-methoxy-10-methylen-2,9-DTD

$C_{11}H_{16}O_5$ Fp.: <0°C
MG: 228.25 $[\alpha]_D^{20}$ : +22,3° (MeOH)

Beispiel 9

Gemäss Beispiel 7, jedoch unter Ersatz des Methanol durch n-Butanol wurde die entsprechende 8β-n-Butoxy-Verbindung hergestellt:

3β-Hydroxymethyl-4α-hydroxy-8β-n-butoxy-10β-methyl-2,9-DTD

$C_{14}H_{24}O_5$ Fp.: <0°C
MG: 272.2 $[\alpha]_D^{20}$ : −40,7° (MeOH)

**Patentansprüche**

1. 3β-Hydroxymethyl-4α-hydroxy-8β-alkoxy-2,9-dioxatricyclo[4,3,1,$0^{3,7}$]decane der allgemeinen Formel I

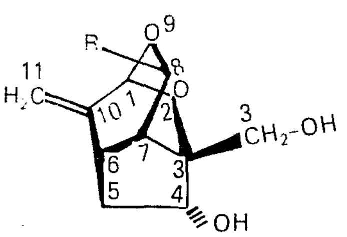

(I)

in der R einen Alkoxyrest mit 1 bis 4 Kohlenstoffatomen bedeutet und die 10,11-Doppelbindung auch zur β-ständigen Methylgruppe hydriert sein kann.

2. 3β-Hydroxymethyl-4α-hydroxy-8β-methoxy-10-methylen-2,9-dioxatricyclo[4,3,1,$0^{3,7}$]decan.

3. 3β-Hydroxymethyl-4α-hydroxy-8β-methoxy-10β-methyl-2,9-dioxatricyclo[4,3,1,$0^{3,7}$]decan.

4. 3β-Hydroxymethyl-4α-hydroxy-8β-äthoxy-10-methylen-2,9-dioxatricyclo[4,3,1,$0^{3,7}$]decan.

5. 3β-Hydroxymethyl-4α-hydroxy-8β-äthoxy-10β-methyl-2,9-dioxatricyclo[4,3,1,$0^{3,7}$]decan.

6. 3β-Hydroxymethyl-4α-hydroxy-8β-butoxy-10β-methyl-2,9-dioxatricyclo [4,3,1,$0^{3,7}$]decan.

7. Verfahren zur Herstellung von 3β-Hydroxymethyl-4α-hydroxy-8β-alkoxy-2,9-dioxatricyclo-[4,3,1,$0^{3,7}$]decane der allgemeinen Formel I, in der R einen Alkoxyrest mit 1 bis 4 Kohlenstoffatomen bedeutet und die 10,11-Doppelbindung auch zur β-ständigen Methylgruppe hydriert sein kann, dadurch gekennzeichnet, dass man Didrovaltratum (1.0) in das entsprechende Acetohydrin (1.1) überführt, dieses mittels Alkohol mit 1 bis 4 Kohlenstoffatomen/p-Toluolsulfonsäure zum 3β-Acetoxymethyl-4β-acetoxy-8β-alkoxy-10-methylen-2,9-dioxatricyclo[4,3,1,$0^{3,7}$]decan (z.B. 1.2) umsetzt, gewünschtenfalls die 10-Methylengruppe zur 10β-Methylgruppe hydriert, die Diacetoxy-Verbindung (z.B. 1.2) zur Dihydroxy-Verbindung (z.B. 1.3) verseift, diese selektiv zur 3β-Hydroxymethyl-4-on-Verbindung (z.B. 1.4) oxidiert und die 4-on-Verbindung selektiv zur 4α-Hydroxy-Verbindung (z.B. 1.5) reduziert.

8. Verfahren nach Anspruch 7, dadurch gekennzeichnet, dass man mit Methanol, Äthanol oder Butanol umsetzt.

9. Verfahren nach Anspruch 7, dadurch gekennzeichnet, dass man mittels Wasserstoff/Raney-Nickel in Gegenwart einer starken anorganischen Base hydriert, wobei gleichzeitig die Diacetoxy-Verbindung zur Dihydroxy-Verbindung verseift wird.

10. Verfahren nach Anspruch 7, dadurch gekennzeichnet, dass man mittels wässriger oder wässrig-alkoholischer Alkalihydroxidlösung oder Kaliumcarbonat/Methanol verseift.

11. Verfahren nach Anspruch 7, dadurch gekennzeichnet, dass man mittels Pyridinchlorochromat oxidiert.

12. Verfahren nach Anspruch 7, dadurch gekennzeichnet, dass man mittels Natriumborhydrid reduziert.

13. Verfahren zur Herstellung von 3β-Hydroxymethyl-4α-hydroxy-8β-alkoxy-2,9-dioxatricyclo-[4,3,1,$0^{3,7}$]decane der allgemeinen Formel I, in der R einen Alkoxyrest mit 1 bis 4 Kohlenstoffatomen bedeutet und die 10,11-Doppelbindung auch zur β-ständigen Methylgruppe hydriert sein kann, dadurch gekennzeichnet, dass man Didrovaltratum (1.0) mittels Alkohol mit 1 bis 4 Kohlenstoffatomen/Halogenwasserstoffsäure zum 3β-Halogenmethyl-4β-acetoxy-8β-alkoxy-10-methylen-2,9-dioxatricyclo[4,3,1,$0^{3,7}$]decan umsetzt (z.B. 2.1), gewünschtenfalls die 10-Methylengruppe zur 10β-Methylgruppe hydriert (z.B. 2.2), die 4β-Acetoxy-Verbindung (z.B. 2.1 oder 2.2) zur 4β-Hydroxyverbindung (z.B. 2.3) verseift, diese selektiv zur 3β-Halogenmethyl-4-on-Verbindung (z.B. 2.4) oxidiert, in dieser die 3β-Halogenmethyl-Gruppe gegen die 3β-Acetoxymethyl-Gruppe austauscht, so dass man die 3β-Acetoxymethyl-4-on-Verbindung (z.B. 2.5) erhält, in dieser selektiv die 4-on-Gruppe zur 4α-Hydroxy-Gruppe reduziert, wobei gleichzeitig die 3β-Acetoxymethyl-Gruppe in die 3β-Hydroxymethyl-Gruppe übergeht, so dass man die 3β-Hydroxymethyl-4α-hydroxy-Verbindung (z.B. 1.5) erhält.

14. Verfahren nach Anspruch 13, dadurch gekennzeichnet, dass man mittels Methanol/Jodwasserstoffsäure, Äthanol/Jodwasserstoffsäure oder Butanol/Jodwasserstoffsäure umsetzt.

15. Verfahren nach Anspruch 13, dadurch gekennzeichnet, dass man mittels Wasserstoff/Platinoxid hydriert.

16. Verfahren nach Anspruch 13, dadurch gekennzeichnet, dass man mittels wässriger oder wässrig-alkoholischer Alkalihydroxidlösung oder

Kaliumcarbonat/Methanol verseift.

17. Verfahren nach Anspruch 13, dadurch gekennzeichnet, dass man mittels Pyridinchlorochromat oxidiert.

18. Verfahren nach Anspruch 13, dadurch gekennzeichnet, dass man mittels Natrium- oder Kaliumacetat in Gegenwart von quartären Alkylammoniumacetaten austauscht.

19. Verfahren nach Anspruch 13, dadurch gekennzeichnet, dass man mittels Lithiumaluminiumhydrid reduziert.

20. Verfahren zur Herstellung von 3β-Hydroxymethyl-4α-hydroxy-8β-alkoxy-2,9-dioxatricyclo-[4,3,1,$0^{3,7}$]decane der allgemeinen Formel I, in der R einen Alkoxyrest mit 1 bis 4 Kohlenstoffatomen bedeutet und die 10,11-Doppelbindung auch zur β-ständigen Methylgruppe hydriert sein kann, dadurch gekennzeichnet, dass man Didrovaltratum (1.0) mittels Alkohol mit 1 bis 4 Kohlenstoffatomen/Halogenwasserstoffsäure zum 3β-Halogenmethyl-4β-acetoxy-8β-alkoxy-10-methylen-2,9-dioxatricyclo[4,3,1,$0^{3,7}$]decan umsetzt (z.B. 2.1), gewünschtenfalls die 10-Methylengruppe zur 10β-Methylgruppe hydriert (z.B. 2.2), die 4β-Acetoxy-Verbindung (z.B. 2.1 oder 2.2) zur 4β-Hydroxyverbindung (z.B. 2.3) verseift, die 4β-Hydroxyfunktion schützt (z.B. 3.1), die 3β-Halogenmethyl-Gruppe gegen die 3β-Acetoxy-Gruppe austauscht und die Schutzgruppe abspaltet, die erhaltene 3β-Acetoxymethyl-4β-hydroxy-Verbindung (z.B. 3.2) selektiv zur 4-on-Verbindung (z.B. 2.5) oxidiert und in dieser selektiv die 4-on-Gruppe zur 4α-Hydroxy-Gruppe reduziert, wobei gleichzeitig die 3β-Acetoxymethyl-Gruppe in die 3β-Hydroxymethyl-Gruppe übergeht, so dass man die 3β-Hydroxymethyl-4α-hydroxy-Verbindung (z.B. 1.5) erhält.

21. Verfahren nach Anspruch 20, dadurch gekennzeichnet, dass man mittels Methanol/Jodwasserstoffsäure, Äthanol/Jodwasserstoffsäure oder Butanol/Jodwasserstoffsäure umsetzt.

22. Verfahren nach Anspruch 20, dadurch gekennzeichnet, dass man mittels Wasserstoff/Platinoxid hydriert.

23. Verfahren nach Anspruch 20, dadurch gekennzeichnet, dass man mittels wässriger oder wässrig-alkoholischer Alkalihydroxidlösung oder Kaliumcarbonat/Methanol verseift.

24. Verfahren nach Anspruch 20, dadurch gekennzeichnet, dass man mittels 3,4-Dihydro-2H-pyran schützt.

25. Verfahren nach Anspruch 20, dadurch gekennzeichnet, dass man mittels Natrium- oder Kaliumacetat in Gegenwart von quartären Alkylammoniumacetaten austauscht.

26. Verfahren nach Anspruch 20, dadurch gekennzeichnet, dass man mittels Jones-Reagenz oxidiert.

27. Verfahren nach Anspruch 20, dadurch gekennzeichnet, dass man mittels Lithiumaluminiumhydrid reduziert.

28. Verfahren zur Herstellung von 3β-Hydroxymethyl-4α-hydroxy-8β-alkoxy-2,9-dioxatricyclo-[4,3,1,$0^{3,7}$]decane der allgemeinen Formel I, in der R einen Alkoxyrest mit 1 bis 4 Kohlenstoffatomen bedeutet und die 10,11-Doppelbindung auch zur β-ständigen Methylgruppe hydriert sein kann, dadurch gekennzeichnet, dass man Didrovaltratum (1.0) mittels Alkohol mit 1 bis 4 Kohlenstoffatomen/Halogenwasserstoffsäure zum 3β-Halogenmethyl-4β-acetoxy-8β-alkoxy-10-methylen-2,9-dioxatricyclo[4,3,1,$0^{3,7}$]decan umsetzt (z.B. 2.1), gewünschtenfalls die 10-Methylengruppe zur 10β-Methylgruppe hydriert (z.B. 2.2), die 4β-Acetoxy-Verbindung (z.B. 2.1 oder 2.2) zur 4β-Hydroxyverbindung (z.B. 2.3) verseift, diese selektiv zur 3β-Halogenmethyl-4-on-Verbindung (z.B. 2.4) oxidiert, die 4-on-Verbindung selektiv zur 4α-Hydroxy-Verbindung (z.B. 4.1) reduziert, diese in die 4α-Acetoxy-Verbindung (z.B. 4.2) umwandelt, in dieser die 3β-Halogenmethylgruppe gegen die 3β-Acetoxymethylgruppe austauscht, so dass man die Diacetoxy-Verbindung (z.B. 4.3) erhält und diese dann zur Dihydroxy-Verbindung (z.B. 1.5) verseift.

29. Verfahren nach Anspruch 28, dadurch gekennzeichnet, dass man mittels Methanol/Jodwasserstoffsäure, Äthanol/Jodwasserstoffsäure oder Butanol/Jodwasserstoffsäure umsetzt.

30. Verfahren nach Anspruch 28, dadurch gekennzeichnet, dass man mittels Wasserstoff/Platinoxid hydriert.

31. Verfahren nach Anspruch 28, dadurch gekennzeichnet, dass man mittels wässriger oder wässrig-alkoholischer Alkalihydroxidlösung oder Kaliumcarbonat/Methanol verseift.

32. Verfahren nach Anspruch 28, dadurch gekennzeichnet, dass man mittels Pyridinchlorochromat oxidiert.

33. Verfahren nach Anspruch 28, dadurch gekennzeichnet, dass man mittels Natriumborhydrid reduziert.

34. Verfahren nach Anspruch 28, dadurch gekennzeichnet, dass man mittels Natrium- oder Kaliumacetat in Gegenwart von quartären Alkylammoniumacetaten austauscht.

**Claims**

1. 3β-Hydroxymethyl-4α-hydroxy-8β-alkoxy-2,9-dioxatricyclo[4,3,1,$0^{3,7}$]decanes of the general formula I

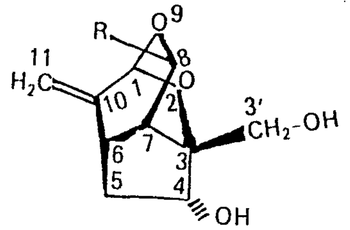

(I)

in which R denotes an alkoxy radical with 1 to 4 carbon atoms and the 10,11-double bond can also be hydrogenated to form a β-oriented methyl group.

2. 3β-Hydroxymethyl-4α-hydroxy-8β-methoxy-10-methylene-2,9-dioxatricyclo-[4,3,1,$0^{3,7}$]decane.

3. 3β-Hydroxymethyl-4α-hydroxy-8β-methoxy-10β-methyl-2,9-dioxatricyclo-[4,3,1,$0^{3,7}$]decane.

4. 3β-Hydroxymethyl-4α-hydroxy-8β-ethoxy-10-methylene-2,9-dioxatricyclo-[4,3,1,$0^{3,7}$]decane.

5. 3β-Hydroxymethyl-4α-hydroxy-8β-ethoxy-10β-methyl-2,9-dioxatricyclo-[4,3,1,$0^{3,7}$]decane.

6. 3β-Hydroxymethyl-4α-hydroxy-8β-butoxy-10β-methyl-2,9-dioxatricyclo[4,3,1,$0^{3,7}$]decane.

7. Process for the preparation of 3β-hydroxymethyl-4α-hydroxy-8β-alkoxy-2,9-dioxatricyclo-[4,3,1,$0^{3,7}$]decanes of the general formula I in which R denotes an alkoxy radical with 1 to 4 carbon atoms and the 10,11-double bond can also be hydrogenated to form a β-oriented methyl group, characterised in that didrovaltrate (1.0) is converted into the corresponding acetohydrin (1.1), the latter is converted by means of alcohol with 1 to 4 carbon atoms/p-toluenesulphonic acid into 3β-acetoxymethyl-4β-acetoxy-8β-alkoxy-10-methylene-2,9-dioxatricyclo[4,3,1,$0^{3,7}$]decane (for example 1.2), if desired the 10-methylene group is hydrogenated to the 10β-methyl group, the diacetoxy compound (for example 1.2) is saponified to the dihydroxy compound (for example 1.3), the latter is selectively oxidised to the 3β-hydroxymethyl-4-one compound (for example 1.4) and the 4-one compound is selectively reduced to the 4α-hydroxy compound (for example 1.5).

8. Process according to Claim 7, characterised in that the reaction is carried out by means of methanol, ethanol or butanol.

9. Process according to Claim 7, characterised in that hydrogenation is carried out by means of hydrogen/Raney nickel in the presence of a strong inorganic base, whereby the diacetoxy compound is saponified at the same time to the dihydroxy compound.

10. Process according to Claim 7, characterised in that saponification is carried out by means of aqueous or aqueous alcoholic alkali metal hydroxyde solution or potassium carbonate/methanol.

11. Process according to Claim 7, characterised in that oxidation is carried out by means of pyridine chlorochromate.

12. Process according to Claim 7, characterised in that reduction is carried out by means of sodium borohydride.

13. Process for the preparation of 3β-hydroxymethyl-4α-hydroxy-8β-alkoxy-2,9-dioxatricyclo-[4,3,1,$0^{3,7}$]decanes of the general formula I in which R denotes an alkoxy radical with 1 to 4 carbon atoms and the 10,11-double bond can also be hydrogenated to form a β-oriented methyl group, characterised in that didrovaltrate (1.0) is converted by means of alcohol with 1 to 4 carbon atoms/hydrogen halide acid into 3β-halomethyl-4β-acetoxy-8β-alkoxy-10-methylene-2,9-dioxatricyclo[4,3,1,$0^{3,7}$]decane (for example 2.1), if desired the 10-methylene group is hydrogenated to the 10β-methyl group (for example 2.2), the 4β-acetoxy compound (for example 2.1 or 2.2) is saponified to the 4β-hydroxy compound (for example 2.3), the latter is selectively oxidised to the 3β-halomethyl-4-one compound (for example 2.4), the 3β-halomethyl group in the latter is replaced by the 3β-acetoxymethyl group, so that the 3β-acetoxymethyl-4-one compound (for example 2.5) is obtained, the 4-one group in the latter is selectively reduced to the 4α-hydroxy group, whereby the 3β-acetoxymethyl group is converted at the same time into the 3β-hydroxymethyl group, so that the 3β-hydroxymethyl-4α-hydroxy compound (for example 1.5) is obtained.

14. Process according to Claim 13, characterised in that the reaction is carried out by means of methanol/hydriodic acid, ethanol/hydriodic acid or butanol/hydriodic acid.

15. Process according to Claim 13, characterised in that hydrogenation is carried out by means of hydrogen/platinum oxide.

16. Process according to Claim 13, characterised in that saponification is carried out by means of aqueous or aqueous alcoholic alkali metal hydroxide solution or potassium carbonate/methanol.

17. Process according to Claim 13, characterised in that oxidation is carried out by means of pyridine chlorochromate.

18. Process according to Claim 13, characterised in that the exchange reaction is carried out by means of sodium or potassium acetate in the presence of quaternary alkylammonium acetates.

19. Process according to Claim 13, characterised in that reduction is carried out by means of lithium aluminium hydride.

20. Process for the preparation of 3β-hydroxymethyl-4α-hydroxy-8β-alkoxy-2,9-dioxatricyclo[4,3,1,$0^{3,7}$]decanes of the formula I in which R denotes an alkoxy radical with 1 to 4 carbon atoms and the 10,11-double bond can also be hydrogenated to form a β-oriented methyl group, characterised in that didrovaltrate (1.0) is converted by means of alcohol with 1 to 4 carbon atoms/hydrogen halide acid into 3β-halomethyl-4β-acetoxy-8β-alkoxy-10-methylene-2,9-dioxatricyclo[4,3,1,$0^{3,7}$]decane (for example 2.1), if desired the 10-methylene group is hydrogenated to the 10β-methyl group (for example 2.2), the 4β-acetoxy compound (for example 2.1 or 2.2) is saponified to the 4β-hydroxy compound (for example 2.3), the 4β-hydroxy function is protected (for example 3.1), the 3β-halomethyl group is replaced by the 3β-acetoxymethyl group and the protective group is split off, the resulting 3β-acetoxymethyl-4β-hydroxy compound (for example 3.2) is selectively oxidised to the 4-one compound (for example 2.5) and the 4-one group in the latter is selectively reduced to the 4α-hydroxy group whereby, the 3β-acetoxymethyl group is converted at the same time into the 3β-hydroxymethyl group, so that the 3β-hydroxymethyl-4α-hydroxy compound (for example 1.5) is obtained.

21. Process according to Claim 20, characterised in that the reaction is carried out by means of methanol/hydriodic acid, ethanol/hydriodic acid

or butanol/hydriodic acid.

22. Process according to Claim 20, characterised in that hydrogenation is carried out by means of hydrogen/platinum oxide.

23. Process according to Claim 20, characterised in that saponification is carried out by means of aqueous or aqueous alcoholic alkali metal hydroxide solution or potassium carbonate/methanol.

24. Process according to Claim 20, characterised in that the protection is effected by means of 3,4-dihydro-2H-pyran.

25. Process according to Claim 20, characterised in that the replacement reaction is carried out by means of sodium or potassium acetate in the presence of quaternary alkylammonium acetates.

26. Process according to Claim 20, characterised in that oxidation is carried out by means of Jones reagent.

27. Process according to Claim 20, characterised in that reduction is carried out by means of lithium aluminium hydride.

28. Process for the preparation of 3β-hydroxymethyl-4α-hydroxy-8β-alkoxy-2,9-dioxatricyclo[4,3,1,0$^{3,7}$]decanes of the general formula I in which R denotes an alkoxy radical with 1 to 4 carbon atoms and the 10,11-double bond can also be hydrogenated to form a β-oriented methyl group, characterised in that didrovaltrate (1.0) is converted by means of alcohol with 1 to 4 carbon atoms/hydrogen halide acid into 3β-halo-methyl-4β-acetoxy-8β-alkoxy-10-methylene-2,9-dioxatricyclo[4,3,1,0$^{3,7}$]decane (for example 2.1), if desired the 10-methylene group is hydrogenated to the 10β-methyl group (for example 2.2), the 4β-acetoxy compound (for example 2.1 or 2.2) is saponified to the 4β-hydroxy compound (for example 2.3), the latter is selectively oxidised to the 3β-halomethyl-4-one compound (for example 2.4), the 4-one compound is selectively reduced to the 4α-hydroxy compound (for example 4.1), the latter is converted into the 4α-acetoxy compound (for example 4.2), the 3β-halomethyl group in the latter is replaced by the 3β-acetoxymethyl group, so that the diacetoxy compound (for example 4.3) is obtained and this is then saponified to the dihydroxy compound (for example 1.5).

29. Process according to Claim 28, characterised in that the reaction is carried out by means of methanol/hydriodic acid, ethanol/hydriodic acid or butanol/hydriodic acid.

30. Process according to Claim 28, characterised in that hydrogenation is carried out by means of hydrogen/platinum oxide.

31. Process according to Claim 28, characterised in that saponification is carried out by means of aqueous or aqueous alcoholic alkali metal hydroxide solution or potassium carbonate/methanol.

32. Process according to Claim 28, characterised in that oxidation is carried out by means of pyridine chlorochromate.

33. Process according to Claim 28, characterised in that reduction is carried out by means of sodium borohydride.

34. Process according to Claim 28, characterised in that the replacement reaction is carried out by means of sodium or potassium acetate in the presence of quaternary alkylammonium acetates.

**Revendications**

1. 3β-hydroxméthyl-4α-hydroxy-8β-alcoxy-2,9-dioxatricyclo[4,3,1,0$^{(3,7)}$]décanes répondant à la formule générale I

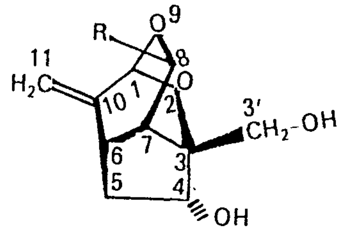

(I)

dans laquelle R représente un reste alcoxy avec 1 à 4 atomes de carbone et la double liaison 10,11 peut également être hydrogénée en le groupe méthyle situé en β.

2. 3β-hydroxyméthyl-4α-hydroxy-8β-méthoxy-10-méthylène-2,9-dioxatricyclo[4,3,1,0$^{(3,7)}$]décane.

3. 3β-hydroxyméthyl-4α-hydroxy-8β-méthoxy-10β-méthyl-2,9-dioxatricyclo[4,3,1,0$^{(3,7)}$]décane.

4. 3β-hydroxyméthyl-4α-hydroxy-8β-éthoxy-10-méthylène-2,9-dioxatricyclo[4,3,1,0$^{(3,7)}$]décane.

5. 3β-hydroxyméthyl-4α-hydroxy-8β-éthoxy-10β-méthyl-2,9-dioxatricyclo[4,3,1,0$^{(3,7)}$]décane.

6. 3β-hydroxyméthyl-4α-hydroxy-8β-butoxy-10β-méthyl-2,9-dioxatricyclo[4,3,1,0$^{(3,7)}$]décane.

7. Procédé pour la préparation de 3β-hydroxyméthyl-4α-hydroxy-8β-alcoxy-2,9-dioxatricyclo[4,3,1,0$^{(3,7)}$]décanes de formule générale I dans laquelle R représente un reste alcoxy avec 1 à 4 atomes de carbone et la double liaison 10,11 peut également être hydrogénée en le groupe méthyle situé en β, caractérisé en ce que l'on convertit du didrovaltratum (1,0) en l'acétohydrine correspondante (1,1), en ce qu'on transforme celle-ci à l'aide d'un alcool avec 1 à 4 atomes de carbone/acide p-toluènesulfonique en 3β-acétoxyméthyl-4β-acétoxy-8β-alcoxy-10-méthylène-2,9-dioxatricyclo[4,3,1,0$^{(3,7)}$]décane (par exemple 1.2) en ce qu'on hydrogène, le cas échéant, le groupe méthylène-10 en le groupe méthyle-10β, on saponifie le composé diacétoxy (par exemple 1.2) en composé dihydroxy (par exemple 1.3), on oxyde celui-ci sélectivement en composé 3β-hydroxyméthyl-4-one (par exemple 1.4) et on réduit sélectivement le composé 4-one en composé 4α-hydroxy (par exemple 1.5).

8. Procédé selon la revendication 7, caractérisé en ce que l'on effectue la réaction avec du méthanol, de l'éthanol ou du butanol.

9. Procédé selon la revendication 7, caractérisé en ce que l'on hydrogène à l'aide d'hydrogène/nickel-Raney en présence d'une base inorganique forte, auquel cas le composé diacétoxy est simultanément saponifié en composé dihydroxy.

10. Procédé selon la revendication 7, caractérisé en ce l'on saponifie au moyen d'une solution aqueuse ou hydro-alcoolique d'un hydroxyde alcalin ou de carbonate de potassium/méthanol.

11. Procédé selon la revendication 7, caractérisé en ce qu'on oxyde au moyen de chloro-chromate de pyridine.

12. Procédé selon la revendication 7, caractérisé en ce qu'on réduit à l'aide de borohydrure de sodium.

13. Procédé pour la préparation de 3β-hydroxyméthyl-4α-hydroxy-8β-alcoxy-2,9-dioxatricyclo[4,3,1,0$^{(3,7)}$]décane de formule générale I, dans laquelle R représente un reste alcoxy avec 1 à 4 atomes de carbone et la double liaison 10,11 peut également être hydrogénée en le groupe méthyle situé en β, caractérisé en ce que l'on convertit du didrovaltratum (1,0) à l'aide d'un alcool avec 1 à 4 atomes de carbone/haloacide en 3β-halométhyl-4β-acétoxy-8β-alcoxy-10-méthylène-2,9-dioxatricyclo[4,3,1,0$^{(3,7)}$]décane (par exemple 2.1), en ce qu'on hydrogène, le cas échéant, le groupe méthylène-10 en le groupe méthyle-10β (par exemple 2.2), on saponifie le composé 4β-acétoxy (par exemple 2.1 ou 2.2) en composé 4β-hydroxy (par exemple 2.3), on oxyde celui-ci sélectivement en composé 3β-halométhyl-4-one (par exemple 2.4), on échange dans celle-ci le groupe 3β-halométhyle contre le groupe 3β-acétoxyméthyle de manière à obtenir le composé 3β-acétoxyméthyl-4-one (par exemple 2.5), on réduit sélectivement dans celle-ci le groupe 4-one en le groupe 4α-hydroxy, auquel cas le groupe 3β-acétoxyméthyle se transforme simultanément en le groupe 3β-hydroxyméthyle, de sorte qu'on obtient le composé 3β-hydroxyméthyl-4-α-hydroxy (par exemple 1.5).

14. Procédé selon la revendication 13, caractérisé en ce qu'on effectue la réaction à l'aide de méthanol/acide iodhydrique, d'éthanol/acide iodhydrique ou de butanol/acide iodhydrique.

15. Procédé selon la revendication 13, caractérisé en ce que l'on hydrogène au moyen d'hydrogène/oxyde de platine.

16. Procédé selon la revendication 13, caractérisé en ce que l'on saponifie au moyen d'une solution aqueuse ou hydro-alcoolique d'un hydroxyde alcalin ou de carbonate de potassium/méthanol.

17. Procédé selon la revendication 13, caractérisé en ce qu'on oxyde au moyen de chloro-chromate de pyridine.

18. Procédé selon la revendication 13, caractérisé en ce qu'on réalise l'échange au moyen d'acétate de sodium ou de potassium en présence d'acétate d'alkylammonium quaternaires.

19. Procédé selon la revendication 13, caractérisé en ce que l'on réduit à l'aide d'hydrure de lithium-aluminium.

20. Procédé pour la préparation de 3β-hydroxyméthyl-4α-hydroxy-8β-alcoxy-2,9-dioxatricyclo[4,3,1,0$^{(3,7)}$]décane de formule générale I, dans laquelle R représente un reste alcoxy avec 1 à 4 atomes de carbone et la double liaison 10,11 peut également être hydrogénée en le groupe méthyle situé en β, caractérisé en ce que l'on convertit du didrovaltratum (1.0) à l'aide d'un alcool avec 1 à 4 atomes de carbone/haloacide en 3β-halométhyl-4β-acétoxy-8β-alcoxy-10-méthylène-2,9-dioxatricyclo[4,3,1,0$^{(3,7)}$]décane (par exemple 2.1), on hydrogène le cas échéant, le groupe méthylène-10 en le groupe méthyle-10β (par exemple 2.2), on saponifie le composé 4β-acétoxy (par exemple 2.1 ou 2.2) en composé 4β-hydroxy (par exemple 2.3), on protège la fonction 4β-hydroxy (par exemple 3.1), on échange le groupe 3β-halométhyle contre le groupe 3β-acétoxyméthyle et on élimine le groupe protecteur, on oxyde sélectivement le composé 3β-acétoxyméthyl-4β-hydroxy (par exemple 3.2) en le composé 4-one (par exemple 2.5) et on réduit sélectivement dans celui-ci le groupe 4-one en le groupe 4α-hydroxy, auquel cas le groupe 3β-acétoxyméthyle se transforme simultanément en le groupe 3β-hydroxyméthyle, de sorte qu'on obtient le composé 3β-hydroxyméthyl-4α-hydroxy (par exemple 1.5).

21. Procédé selon la revendication 20, caractérisé en ce qu'on effectue la réaction à l'aide de méthanol/acide iodhydrique, d'éthanol/acide iodhydrique, ou de butanol/acide iodhydrique.

22. Procédé selon la revendication 20, caractérisé en ce que l'on hydrogène au moyen d'hydrogène/oxyde de platine.

23. Procédé selon la revendication 20, caractérisé en ce que l'on saponifie au moyen d'une solution aqueuse ou hydro-alcoolique d'un hydroxyde alcalin ou de carbonate de potassium/méthanol.

24. Procédé selon la revendication 20, caractérisé en ce que l'on protège à l'aide de 3,4-dihydro-2H-pyranne.

25. Procédé selon la revendication 20, caractérisé en ce qu'on réalise l'échange au moyen d'acétate de sodium ou de potassium en présence d'acétates d'alkylammonium quaternaires.

26. Procédé selon la revendication 20, caractérisé en ce qu'on oxyde au moyen du réactif Jones.

27. Procédé selon la revendication 20, caractérisé en ce que l'on réduit à l'aide d'hydrure de lithium-aluminium.

28. Procédé pour la préparation de 3β-hydroxyméthyl-4α-hydroxy-8β-alcoxy-2,9-dioxatricyclo[4,3,1,0$^{(3,7)}$]décane de formule générale I, dans laquelle R représente un reste alcoxy avec 1 à 4 atomes de carbone et la double liaison 10,11 peut également être hydrogénée en le groupe méthyle situé en β, caractérisé en ce que l'on convertit du didrovaltratum (1.0) à l'aide d'un alcool avec 1 à 4 atomes de carbone/haloacide en 3β-halométhyl-4β-acétoxy-8β-alcoxy-10-méthylène-2,9-dioxatricyclo[4,3,1,0$^{(3,7)}$]décane (par exemple 2.1), en ce qu'on hydrogène le cas échéant, le groupe méthylène-10 en le groupe méthyle-10β (par exemple 2.2), on saponifie le composé 4β-acétoxy (par exemple 2.1 ou 2.2) en composé 4β-hydroxy (par exemple 2.3), on oxyde celui-ci sélectivement en composé 3β-halométhyl-4-one (par exemple 2.4), on réduit sélectivement le composé 4-one en le composé 4α-hydroxy (par exemple 4.1), on transforme celui-ci en le composé 4α-acétoxy (par exemple 4.2), on échange dans celui-ci le groupe 3β-halométhyle contre le groupe 3β-acétoxyméthyle, de sorte qu'on obtient le composé diacétoxy (par exemple 4.3) et on saponifie ensuite celui-ci en le composé dihydroxy (par exemple 1.5).

29. Procédé selon la revendication 28, caractérisé en ce qu'on effectue la réaction à l'aide de méthanol/acide iodhydrique, d'éthanol/acide iodhydrique ou de butanol/acide iodhydrique.

30. Procédé selon la revendication 28, caractérisé en ce que l'on hydrogène au moyen d'hydrogène/oxyde de platine.

31. Procédé selon la revendication 28, caractérisé en ce que l'on saponifie au moyen d'une solution aqueuse ou hydro-alcoolique d'un hydroxyde alcalin ou de carbonate de potassium/méthanol.

32. Procédé selon la revendication 28, caractérisé en ce qu'on oxyde au moyen de chloro-chromate de pyridine.

33. Procédé selon la revendication 28, caractérisé en ce qu'on réduit à l'aide de borohydrure de sodium.

34. Procédé selon la revendication 28, caractérisé en ce qu'on réalise l'échange au moyen d'acétate de sodium ou de potassium en présence d'acétates d'alkylammonium quaternaires.